Europäisches Patentamt

European Patent Office

Office européen des brevets

(19)

(11) Publication number: **0 176 432**
**B1**

(12)

# EUROPEAN PATENT SPECIFICATION

(45) Date of publication of patent specification: **14.09.88**

(21) Application number: **85401807.4**

(22) Date of filing: **18.09.85**

(51) Int. Cl.⁴: **B 01 J 27/10,** C 07 C 19/045,
C 07 C 17/156

(54) Method for preparing a catalyst for the synthesis of 1,2-dichloroethane.

(30) Priority: **19.09.84 IT 2271784**

(43) Date of publication of application:
**02.04.86 Bulletin 86/14**

(45) Publication of the grant of the patent:
**14.09.88 Bulletin 88/37**

(84) Designated Contracting States:
**BE DE FR GB IT NL**

(56) References cited:
**GB-A-1 345 653**
**US-A-3 624 170**
**US-A-4 460 699**
**US-A-4 495 308**

(73) Proprietor: **AUSIMONT S.p.A.**
**31, Foro Buonaparte**
**I-20121 Milano (IT)**

(72) Inventor: **Cavaterra, Enrico**
**21 via 4 Novembre**
**Saronno Varese (IT)**
Inventor: **Bossi, Alessandro**
**10 Via Mantegna**
**I-28100 Novara (IT)**

(74) Representative: **Hirsch, Marc-Roger**
**Cabinet Hirsch 34 rue de Bassano**
**F-75008 Paris (FR)**

EP 0 176 432 B1

Courier Press, Leamington Spa, England.

## Description

This invention relates to a method for preparing a catalyst for the synthesis of 1,2-dichloroethane by oxychlorination of $C_2H_4$ within a fluidized bed, said catalyst containing from 1 to 10% by weight of Cu, from 0.4 to 1.2 mole of magnesium per mole of Cu and a carrier consisting of $Al_2O_3$.

Background of the invention
Field of the invention
Catalysts of the aforesaid type have been described, as well as the respective methods of preparing them, in several patents. See, for instance, British patent 971 996, US—A—4 125 534, British patent 1 345 653 and USP 4 587 230 of Enrico Cavaterra et al, filed March 21, 1985 (equivalent to EP—A—0119933) which disclose the impregnation of alumina with solutions of $CuCl_2$ and $MgCl_2$.

In the first cases, however, no precise enough direction is given to obtain the high yields required by modern plants and, at the same time, a satisfactory degree of fluidization.

These results, on the other hand, can be obtained by using the catalyst according to the aforesaid Cavaterra et al application. However, in this case, there are also drawbacks; in fact, said application teaches that excellent performances can be achieved only if the catalyst is treated with hydrochloric acid (HCl), which may involve problems concerning ecology and corrosion in the plant, during the catalyst preparation.

Description of the prior art
It has now been surprisingly found that the same excellent performances as those achieved according to the Cavaterra et al application can be achieved without using HCl during preparation of the catalyst, provided $CuCl_2$ and $MgCl_2$ are added at the same time and provided the surface area of the carrier is comprised between 80 and 170 $m^2/g$. In other words, it has been observed that, when the surface area of the carrier has a value comprised within said given range, (and provided the amount of magnesium chloride is sufficiently high), the magnesium chloride acts as regulator of the copper on the surface, a function which had previously been ascribed to HCl.

The invention relates to a method for preparing a catalyst for the synthesis of 1,2-dichloroethane by oxychlorination of $C_2H_4$ within a fluidized bed, said catalyst containing from 1 to 10% by weight of Cu, from 0.4 to 1.2 mole of magnesium per mole of Cu and a carrier consisting of $Al_2O_3$, said method being characterized in that the carrier presenting a surface area ranging from 80 to 170 $m^2/g$ is impregnated according to the dry impregnation technique with an aqueous solution containing both $CuCl_2$ and $MgCl_2$, wherein the molar ratio:

$$X = \frac{\text{Al on the surface}}{\text{Cu on the surface}}$$

determined according to the technique known as X-ray photoemission spectroscopy, is at least 40% higher than the molar ratio:

$$Y = \frac{\text{Total Al present within the catalyst}}{\text{Total Cu present within the catalyst}}$$

the ratio W=X/Y being therefore ≥1.40 and in that the catalyst thus obtained is dried and activated.
According to a preferred embodiment of the invention:
— the catalyst contains from 3 to 6% by weight of copper;
— said X ratio is equal to or higher than 40:1;
— the amount of magnesium in the catalyst (and in the solution) is comprised between 0.5 and 1.1 moles per mole of copper.
Said molar ratio:

$$X = \frac{\text{Al on the surface}}{\text{Cu on the surface}}$$

must be determined according to the technique known as XPS analysis (described hereinafter), that supplies the data pertinent to a superficial micro-layer having a thickness comprised between 2 and 3 nm (20—30 Ångstrom). Generally, according to the method of the invention, the copper concentration on the surface has to be zero or, in any event, much lower than the copper concentration within the layers directly underlying the superficial layer, said superficial layer having substantially a thickness from 2 to 3 nm.

The catalysts according to the invention display excellent fluidization features and maintain high HCl conversions, as well as in the case of high $HCl/C_2H_4$ ratios, so that high dichloroethane (DCE) yields with respect to $C_2H_4$ are achieved.

In the catalysts according to the invention, considering the single granule, the Cu lies prevailingly inside the pores, i.e. it is laid on the inner surface of same; such proportion of Cu, although when involved in the conversion cycles and in the equilibria among the various forms ($Cu_2Cl_2$, $Cu_2Cl_4$, etc.), depending on the $HCl/C_2H_4$ feed ratio, it does not give place to the sticking phenomenon, that, on the contrary, affects the zones of possible contact among the different granules.

The outstanding feature of the present catalysts is that, in practice, the active part is almost completely segregated inside the pores of the carrier, the Cu concentration on the outer surface being very slight and in any event clearly lower than the Cu concentration on the outer surface which is a disadvantage of the conventional catalysts, as shown by measurements carried out by means of the XPS technique. Furthermore, it remains the same after long periods of time.

By using these catalysts, the reaction within a fluidized bed can be carried out without any adverse effect on the fluidization, using high $HCl/C_2H_4$ ratios in the feed and with high HCl conversions, so that high yields of DCE are obtained with respect to ethylene. The conditions of use of these catalysts do not substantially differ from those of the catalysts previously described in the art. $C_2H_4$, HCl and a gas containing $O_2$ (generally air) are fed into a gaseous phase, pre-heated to a temperature close to, but not higher than, the reaction temperature, the latter comprised between 200 and 250°C, preferably between 220 and 235°C. The other operating parameters are generally comprised within the following ranges:

(A) Air/ethylene ratio: it must be such that the $O_2$ content, in the gaseous exhausts, after condensation of DCE, $H_2O$ and HCl, is comprised between 3 and 10% by volume.

(B) HCl/ethylene ratio: it must as close as possible to the stoichiometric value (2/1 molar) compatible with the maintenance of good fluidization conditions of the catalytic bed and of a sufficiently high conversion of HCl, conditions which depend, as already noted, on the specific catalyst.

(C) Contact time (expressed as a ratio of the volume of the catalytic bed in a fluidized state to the volumetric flow of the reactant mixture, under the temperature and pressure conditions existing in the catalytic bed); it depends essentially on the specific type of catalyst utilized; generally it ranges between 10 and 40 seconds, preferably between 20 and 30 seconds.

(D) Linear velocity of the gases (expressed in cm/s): it is chosen within the range between the rate of a minimum fluidization and the carrying along rate, both being typical for the type of catalyst; generally, said rate is comprised between 10 and 50 cm/s, and preferably between 20 and 40 cm/s.

(E) Total pressure during the reaction (important for achieving an effective contact among the reactants, in a gaseous phase, and the catalyst, in a solid phase): generally, pressures used are higher than atmospheric pressure, up to 6 bars; at higher pressures energy waste becomes predominant, due to the compression work. The following examples are given by way of non-limitative illustration.

Operating conditions common to the different examples

Measurement of the outer surface concentration of Cu was carried out by means of the XPS technique (see C. D. Wagner: Handbook of X-Ray Photoemission Spectroscopy; Perkin Elmer Co., Eden Prairie; 1979) based on X-ray irradiation and on the measurement of the energy level and of the energy intensity of the electrons emitted by the solid. The energy level of such electrons is characteristic of the element and the energy intensity is proportional to the number of atoms present in the volume of sample, down to a depth substantially of 2 to 3 nm (20—30 Å) from the surface. As the average size of the catalysts is about 50 micrometers (in literature values from 20 to 80 nm are usually cited), the measurement value of the atomic concentrations refers to about 1 ten-thousandth of the granule diameter, namely, essentially to its outer surface. In detail, a small amount of a sample (a few milligrams) was pressed onto a small plate of pure indium in order to obtain an analyzable surface having an area equal to several square nanometers; the samples were then analyzed under a high-pushed vacuum at a basic pressure of $2.10^7$ Pa, using an X-ray source working at 400 W and fitteduwith a Mg anode (Kα radiation of magnesium). The photoemission spectra of the present elements, i.e. 0/1s, Cl/2p, Mg/2p, Al/2p, were gathered under conditions of high resolution by using a computer for the digitalized acquisition of the data, with a maximation of the signal/noise ratio. After removal of the background noise, the areas of the photoemission peaks were calculated by means of numerical integration; the intensity value thus obtained, corrected for the respective sensitivity factor, was directly proportional to the surface atomic concentration of the respective element.

All catalysts were prepared by means of the "dry impregnation" technique, described for instance by A. V. Neimark, L. I. Kheifez and V. B. Fenelonov on Ind. Eng. Chem. Prod. Res. Dev. 1981, 20, page 441.

Example 1—Preparation of the catalyst by means of a "dry impregnation"

A microspheroidal alumina having a surface area of 170 sq.m/g and an average particle diameter of 50 nm was chosen as carrier and the volume of the impregnating solution was equal to the volume of the alumina pores. Said solution contained:

— $CuCl_2$, in an amount such that the final catalyst contained 4.16% by weight of Cu;

— 0.736 moles of $MgCl_2$ per mole of Cu.

In further detail, the operating conditions described were as described hereinafter:

(A) 10.5 l of deionized water were heated to 80°C and 6.083 kg of $CuCl_2 \cdot 2H_2O$; 5,291 kg of $MgCl_2 \cdot 6H_2O$ were then added, under stirring.

3

Heating and stirring were continued until a complete dissolution was achieved and the solution thus obtained was then cooled to 25°C;

(B) 43.0 kg of said alumina were put into a rotary container and the solution obtained according to (A) was slowly sprayed, over a period of 1 hour, onto the alumina, kept under stirring by the rotation of the container, taking care to avoid the formation of clots. Afterwards, the temperature was raised to 150°C, with a gradient of 25°C/h and said temperature was kept at 150°C for a further 3 hours. The catalyst was then slowly cooled to 40°C, keeping the container under constant rotation. The analysis gave the results shown in Table 1.

Examples 2, 3, 4 (behavior of the catalyst)

The catalyst of Example 1 was introduced into a glass reactor having a diameter of 4 cm and a height of 3 m, capable of withstanding pressures up to 6 bars. Said catalyst was then activated in situ at 180°C for 4 hours in the air. The thus activated catalyst was tested in an oxychlorination of $C_2H_4$ within a fluidized bed at a pressure of 4 bars (absolute), according to an air/$C_2H_4$ molar ratio equal to 3.2 and with a contact time of 28 seconds; data and results obtained are shown in Table 2.

Example 5 (Comparative)

Example 1 was repeated but without any addition of Mg; the results obtained are shown in Table 1.

Examples 6 and 7 (Comparative)

Examples 2 and 3 were repeated, but the catalyst was replaced by the product lacking in magnesium, prepared according to Example 5. Data and results obtained are shown in Table 2. All these examples show that the lower concentrations of Cu on ghe surface (corresponding to higher Al/Cu ratios) lead to better fluidization characteristics of the catalyst, which allows to work with higher HCl/$C_2H_4$ feed ratios and thus to obtain higher yields in dichloroethane.

TABLE 1

| Examples | % of atoms on the surface | | | $X=\dfrac{\text{Al on the surface}}{\text{Cu on the surface}}$ | $Y=\dfrac{\text{Total Al}}{\text{Total Cu}}$ | $W=\dfrac{X}{Y}$ |
|---|---|---|---|---|---|---|
| | Cu | Cl | Al | | | |
| 1 | 2.1 | 7.7 | 90.2 | 42.9 | 25.3 | 1.69 |
| 5(*) | 3.7 | 7.7 | 88.6 | 24.1 | 26.7 | 0.90 |

(*) Comparative.

4

TABLE 2

| Example | $\dfrac{Al}{Cu}$ | Activation T (°C) | Oxychlorination T (°C) | $HCl/C_2H_4$ (by moles) | DCE yield (molar % on fed $C_2H_4$) | HCl Conversion (%) | Fluidization |
|---------|------|------------|----------------|-----------|----------------|------------|--------------|
| 2 | 42 | 180 | 225 | 1.824 | 91.1 | 99.9 | Excellent |
| 3 | 42 | 180 | 225 | 1.946 | 96.6 | 99.3 | Excellent |
| 4 | 42 | 180 | 225 | 1.990 | 97.7 | 98.2 | Excellent |
| 6 | 24.1 | 180 | 225 | 1.816 | 90.7 | 99.9 | Good |
| 7 | 24.1 | 180 | 225 | 1.926 | 95.7 | 99.4 | Bad |

**Claims**

1. A method for preparing a catalyst for the synthesis of 1,2-dichloroethane by oxychlorination of $C_2H_4$ within a fluidized bed, said catalyst containing from 1 to 10% by weight of Cu, from 0.4 to 1.2 mole of magnesium per mole of Cu and a carrier consisting of $Al_2O_3$, said method being characterized in that the carrier presenting a surface area ranging from 80 to 170 $m^2/g$ is impregnated according to the dry impregnation technique with an aqueous solution containing both $CuCl_2$ and $MgCl_2$, wherein the molar ratio:

$$X = \frac{Al \text{ on the surface}}{Cu \text{ on the surface}}$$

determined according to the technique known as X-ray photoemission spectroscopy, is at least 40% higher than the molar ratio:

$$Y = \frac{Total \text{ Al present within the catalyst}}{Total \text{ Cu present within the catalyst}}$$

the ratio $W = X/Y$ being therefore $\geq 1.40$ and in that the catalyst thus obtained is dried and activated.

2. The method of claim 1, wherein said catalyst contains from 3 to 6% by weight of Cu.

3. The method of claim 1, wherein the ratio X is equal to or higher than 40.

4. The method of claim 1, wherein the copper concentration on the outer surface of the carrier is zero or at least much lower than the copper concentration in the layers directly underlying the superficial layer, said superficial layer having substantially a thickness from 2 to 3 nm.

5. The method of claim 1, wherein the amount of magnesium in the catalyst is comprised inclusively between from 0.5 to 1.1 moles per mole of copper.

**Patentansprüche**

1. Verfahren zur Herstellung eines Katalysators für die Synthese von 1,2-Dichloräthan durch Oxychlorierung von $C_2H_4$ in einem Flüssigkeitsbett, wobei besagter Katalysator 1 bis 10 Gew.-% Cu, 0,4 bis 1,2 Mol Magnesium auf 1 Mol Cu und einen aus $Al_2O_3$ bestehenden Träger enthält, dadurch gekennzeichnet, dass der Träger, der eine Oberfläche von 80 bis 170 $m^2/g$ aufweist, gemäss der Trocken-Impregnierungs-Technik mit einer wässrigen, $CuCl_2$ und $MgCl_2$ enthaltenden, Lösung imprägniert wird, wobei das molare Verhältnis:

$$X = \frac{Al \text{ auf der Oberfläche}}{Cu \text{ auf der Oberfläche,}},$$

das gemäss der als Röntgen-Photoemissions-Spektroskopie bekannten Technik bestimmt wird, um mindestens 40% höher ist als das molare Verhältnis:

$$Y = \frac{Gesamtes \text{ im Katalysator vorhandenes Al}}{Gesamtes \text{ im Katalysator vorhandenes Cu,}}$$

das Verhältnis $W = X/Y$ deshalb $\geq 1,40$ ist und dass der auf diese Weise erhaltene Katalysator getrocknet und aktiviert wird.

2. Verfahren gemäss Anspruch 1, dadurch gekennzeichnet, dass der Katalysator 3 bis 6 Gew.-% Cu enthält.

3. Verfahren gemäss Anspruch 1, dadurch gekennzeichnet, dass das Verhältnis X gleich oder grösser als 40 ist.

4. Verfahren gemäss Anspruch 1, dadurch gekennzeichnet, dass der Kupfergehalt auf der äusseren Oberfläche des Trägers gleich null oder zumindest wesentlich niedriger ist, als der Kupfergehalt der Schichten direkt unterhalb der Oberflächenschicht, wobei die letztere eine Dicke von 2 bis 3 nm aufweist.

5. Verfahren gemäss Anspruch 1, dadurch gekennzeichnet, dass der Katalysator zwischen 0,5 une 1,1 Mol Magnesium auf 1 Mol Kupfer enthält.

**Revendications**

1. Un procédé de préparation d'un catalyseur pour la synthèse du 1,2-dichloroéthane par oxychloruration de $C_2H_4$ au sein d'un lit fluidisé, ce catalyseur contenant de 1 à 10% en poids de Cu, de 0,4 à 1,2 moles de magnésium par mole de Cu et un support constitué d'Al$_2$O$_3$, ce procédé étant caractérisé en ce que le support, présentant une surface spécifique comprise entre 80 et 170 m$^2$/g, est imprégné selon la technique d'imprégnation à sec l'aide d'une solution aqueuse contenant à la fois CuCl$_2$ et MgCl$_2$, dans laquelle le rapport molaire:

$$X=\frac{\text{Al en surface}}{\text{Cu en surface}}$$

déterminé selon la technique connue sous le nom de spectroscopie de photo-émission aux rayons X et supérieur d'au moins 40% au rapport molaire:

$$Y=\frac{\text{Al total présent au sein du catalyseur}}{\text{Cu total présent au sein du catalyseur}}$$

le rapport W=X/Y étant donc ≥1,40 et en ce que le catalyseur ainsi obtenu est séché et activé.

2. Le procédé selon la revendication 1, dans lequel ce catalyseur contient de 3 à 6% de Cu.

3. Le procédé selon la revendication 1, dans lequel le rapport X est égal ou supérieur à 40.

4. Le procédé selon la revendication 1, dans lequel la concentration en cuivre à la surface extérieure du support est égale à zéro ou au moins de beaucoup inférieure à la concentration en cuivre dans les couches directement sous-jacentes à la couche superficielle, l'épaisseur de cette couche superficielle étant sensiblement de 2 à 3 nm.

5. Le procédé selon la revendication 1 dans lequel la quantité de magnésium dans le catalyseur est comprise inclusivement entre 0,5 et 1,1 moles par mole de cuivre.